**Europäisches Patentamt**

**European Patent Office**

**Office européen des brevets**

(11) Numéro de publication : **0 145 553 B1**

(12) **FASCICULE DE BREVET EUROPÉEN**

(45) Date de publication du fascicule du brevet :
04.05.88

(51) Int. Cl.⁴ : **C 07 C102/08**

(21) Numéro de dépôt : **84402294.7**

(22) Date de dépôt : **13.11.84**

(54) Procédé de préparation d'un catalyseur pour l'hydratation des nitriles et procédé d'hydratation de l'acrylonitrile en acrylamide.

(30) Priorité : **17.11.83 BR 8306403**

(43) Date de publication de la demande :
**19.06.85 Bulletin 85/25**

(45) Mention de la délivrance du brevet :
**04.05.88 Bulletin 88/18**

(84) Etats contractants désignés :
**AT DE FR GB IT NL**

(56) Documents cités :
**US-A- 3 929 881
US-A- 3 939 205
CHEMISTRY LETTERS, no. 7, juillet 1983, pages 1047-1050, The Chemical Society of Japan; H. HIRAI et al.:
"Polymer-protected copper colloids as catalysts for selective hydration of acrylonitrile"
CHEMICAL ABSTRACTS, vol. 98, no. 4, 24 janvier 1983, page 8, abrégé no. 17179m, Columbus, Ohio, US; & JP - A - 57 130 959 (ASAHI CHEMICAL INDUS-TRY CO., LTD.) 13-08-1982
CHEMICAL ABSTRACTS, vol. 99, no. 2, 11 juillet 1983, page 10, abrégé no. 6197k, Columbus, Ohio, US; & JP - A - 58 52 255 (ASAHI CHEMICAL INDUSTRY CO., LTD.) 28-03-1983**

(73) Titulaire : **RHONE-POULENC CHIMIE
25, quai Paul Doumer
F-92408 Courbevoie Cédex (FR)**

(72) Inventeur : **Salvi, Franco
520, rue Eng. Humberto Soares de Camargo
Campinas Etat de Sao Paulo (BR)**

(74) Mandataire : **Fabre, Madeleine-France et al
RHONE-POULENC INTERSERVICES Service Brevets
Chimie 25, quai Paul Doumer
F-92408 Courbevoie Cédex (FR)**

# 0 145 553

## Description

La présente invention a pour objet un procédé de préparation d'un catalyseur à base de cuivre et de vanadium pour l'hydratation des nitriles ainsi que le procédé d'hydratation de l'acrylonitrile en acrylamide en présence dudit catalyseur.

Des amides insaturés tels que l'acrylamide et le méthacrylamide sont préparés industriellement par hydratation catalytique des nitriles correspondants. Des catalyseurs comme le cuivre métallique et des compositions à base de ce métal dans ses divers états d'oxydation avec d'autres métaux, notamment ceux de transition, sont déjà connus.

Un des problèmes présentés par les catalyseurs à base de cuivre est leur court temps de vie utile. En effet, le brevet français 2 159 780 pose déjà le problème de la perte considérable de l'activité de ces catalyseurs après quelques heures d'utilisation ; il décrit un procédé en deux étapes pour récupérer l'activité initiale de ces catalyseurs.

Il a été proposé dans les demandes japonaises n° 57.130959 et 58.52255 d'utiliser pour hydrater l'acrylonitrile en acrylamide des catalyseurs obtenus par réduction de complexes de cuivre II en dispersion aqueuse. La préparation de tels complexes nécessite une opération de chauffage soit lors de leur synthèse soit lors de leur séparation du milieu de synthèse.

Un autre problème lié à l'emploi de compositions catalytiques à base de cuivre pour l'hydratation de nitriles est celui de la sélectivité pour l'obtention du produit désiré. On a vérifié que certains métaux de transition, notamment le vanadium, confèrent, à côté d'une augmentation de la vie utile des catalyseurs à base de cuivre, une meilleure performance du point de vue de l'activité et de la sélectivité quant à l'hydratation de nitriles. Un exemple est donné dans la demande de brevet japonais 77 95608. Cependant, la préparation du catalyseur selon cette demande présente quelques inconvénients : comme elle est fondée sur la décomposition thermique de complexes de cuivre, elle utilise donc des températures élevées.

Elle utilise aussi des composés ayant la structure de la pyridine, de manipulation incommode ainsi que des solvants comme le dioxanne ce qui augmente le nombre d'étapes nécessaires.

Le brevet américain US 3 939 205 mentionne la préparation de catalyseurs à base de cuivre et de vanadium, la réduction du cuivre étant faite à l'aide du formaldéhyde en milieu basique. Les inconvénients présentés sont l'utilisation d'un grand excès de réducteur et l'exigence de températures de l'ordre de 90 °C. D'autre part, les phases de réduction et de neutralisation ainsi que la préparation des solutions nécessaires augmentent le nombre total d'étapes.

Une autre voie (brevet américain n° 3.929 881) consiste à décomposer des hydrures de cuivre. Parmi les inconvénients de ce procédé, on trouve d'abord, le fait selon lequel l'hydrure de cuivre à décomposer est instable ; ainsi, il doit être préalablement préparé par réduction en milieu acide de composés de cuivre. Ensuite, la décomposition des hydrures exige l'emploi d'une température élevée (si elle se fait par voie sèche) ou d'une température de bases fortes (si elle se fait par voie humide). Par ailleurs, il faut signaler encore l'emploi éventuel de solvants ou d'amides organiques contribuant à augmenter le nombre total d'étapes.

Le brevet américain US 4 096 184 décrit des catalyseurs préparés par traitement de vanadates avec des sels cuivreux ou avec au moins deux membres d'un groupe constitué par le cuivre métallique, des sels cuivreux ou des sels cuivriques. Ce brevet présente aussi quelques inconvénients.

On constate d'abord, que selon la plupart des exemples de réalisation, on ne sépare pas le catalyeur fabriqué de la masse réactionnelle utilisée pour le préparer. Il en résulte que pour hydrater les nitriles en présence de ces catalyseurs, de grosses quantités de catalyseurs sont nécessaires (d'après les exemples, de 20 à 57 % en poids par rapport à l'acrylonitrile à hydrater). Un autre inconvénient apparaît dans les cas où le catalyseur est préparé et séparé pour la 2e phase (l'hydratation), comme on peut le constater dans les exemples 39 à 42 du brevet. Dans de telles situations, à côté des catalyseurs déjà préparés (étapes « A » des exemples), il faut additionner du cuivre métallique, c'est-à-dire former des catalyseurs mixtes car, utilisés seuls, les catalyseurs préparés dans les phases « A » sont peu efficaces (voir l'exemple 43 où le cuivre n'est pas utilisé), surtout quand on utilise des solutions diluées dans le nitrile.

La Demanderesse a trouvé un procédé de préparation d'un catalyseur à base de cuivre et de vanadium particulièrement efficace, sélectif et présentant une longue durée de vie utile.

Le procédé faisant l'objet de l'invention est caractérisé en ce qu'il est réalisé à température ambiante sous atmosphère inerte et comporte les étapes suivantes :

a) mise en solution dans l'eau d'un agent complexant susceptible de complexer sous forme hydrosoluble les composés de cuivre II ;

b) alcalinisation de la solution obtenue par un hydroxyde de métal alcalin ;

c) mise en solution dans le milieu obtenu à l'étape b) d'un composé du cuivre II ;

d) mise en solution dans le milieu obtenu à l'étape c) d'un composé hydrosoluble du vanadium ;

e) mise en solution dans le milieu à l'étape d) d'un agent réducteur des ions cuivre II ;

f) et récupération du catalyseur formé après lavage du précipité obtenu ; les quantités de réactifs mises en œuvres aux étapes a), b), c), d) et e) correspondant à :

— un rapport molaire agent complexant/composé du cuivre II allant de 1/1 à 10/1 ;

— un rapport molaire base/composé de cuivre II allant de 2/1 à 10/1 ;

— un rapport molaire cuivre II/vanadium d'au moins 6/1 ;

— une quantité d'agent réducteur allant de 100 % à 300 % de la quantité stoechiométrique.

Selon l'invention le catalyseur est préparé sous atmosphère inerte afin d'obtenir et de préserver dès le début de la fabrication une haute réactivité et un temps de vie utile long dudit catalyseur. Cette atmosphère inerte, non oxydante peut être obtenue, par exemple, à l'aide d'un courant d'azote passé à l'intérieur du réacteur de préparation. Cette même atmosphère inerte devra être avantageusement préservée pendant la conservation du catalyseur fini, pour les mêmes raisons.

L'agent complexant utilisé dans la première étape a) est indispensable pour complexer les ions de cuivre II en les gardant stables et en solution jusqu'à l'étape de réduction. Des agents tels que l'acide éthylènediaminetétracétique (EDTA), le tartrate double de sodium et de potassium, le chlorhydrate d'orthophénantroline, l'hexamétaphosphate de sodium, le citrate de sodium, la cuproine... peuvent être utilisés. D'une façon préférentielle, on utilise l'EDTA et tout particulièrement le tartrate double de sodium et de potassium.

Le composé de cuivre II mis en œuvre à l'étape c) peut être sélectionné parmi les membres du groupe qui comprend le chlorure de cuivre II dihydraté, le nitrate de cuivre II hexahydraté, le sulfate de cuivre II pentahydraté et l'hydroxyde cuivreux. Préférentiellement, on utilise le sulfate de cuivre II pentahydraté ou l'hydroxyde cuivreux.

L'agent d'alcalinisation à utiliser à l'étape b) peut être un hydroxyde de métal alcalin. Un mode préférentiel de réalisation de l'invention consiste à utiliser l'hydroxyde de sodium.

Le composé de vanadium à solubiliser dans le milieu réactionnel obtenu à l'étape c) peut être choisi parmi les membres du groupe constitué par le pentoxyde de vanadium, le vanadate d'ammonium et le sulfate de vanadyle. Une réalisation très favorable de l'invention est celle qui utilise le pentoxyde de vanadium.

L'étape e) de réduction des ions cuivre II peut être réalisée à l'aide de réducteurs tels que le borohydrure de sodium, l'hydrazine, l'hypophosphite de sodium et le chlorhydrate d'hydroxylamine. Préférentiellement, on utilise l'hydrazine et le chlorhydrate d'hydroxylamine.

L'eau utilisée est celle soumise à des traitements normaux dans un laboratoire, comme l'eau distillée, l'eau bidistillée, l'eau déminéralisée, désaérées. Dans les exemples ci-après on a utilisé l'eau déminéralisée par commodité.

Lorsque l'agent complexant utilisé est du tartrate double de sodium et de potassium, le rapport molaire complexant/cuivre se situe entre 1 : 1 et 3 : 1 de préférence.

La base mise en œuvre pour alcaliniser le milieu à l'étape b) peut être introduite telle quelle ou bien sous forme d'une solution aqueuse existant sur le marché ou préparée au préalable. Dans ce dernier cas, les solutions d'hydroxyde de sodium à 5 %-50 % en poids répondent parfaitement aux objectifs de l'invention.

Pour une bonne réalisation de l'invention les composés de cuivre II et de vanadium peuvent être mis en œuvre selon des quantités telles que le rapport atomique Cu/V soit compris entre 6 : 1 et jusqu'à quelques centaines d'atomes-gramme de cuivre par atome-gramme de vanadium. Préférentiellement, on utilise des rapports atomiques Cu/V compris entre 16 : 1 et 200 : 1.

Les quantités des divers réducteurs utilisables pour l'étape de réduction du cuivre peuvent varier dans de larges limites, le rapport entre le réducteur et le cuivre à réduire variant de 100 % jusqu'à 300 % de la quantité stoechiométrique demandée.

Toutefois, il est intéressant de remarquer que, si le choix d'un réducteur particulier est indifférent du point de vue de la réalisation de l'invention, celui-ci peut par contre avoir une influence sur l'activité du catalyseur obtenu. En effet, les catalyseurs obtenus par la réduction du cuivre II à l'aide de l'hydrazine présentant une activité plus élevée dans la conversion de l'acrylonitrile en acrylamide que ceux obtenus par réduction à l'aide du chlorhydrate d'hydroxylamine.

La quantité de réducteur à mettre en œuvre est également conditionnée par le choix de celui-ci en vue de l'activité recherchée du catalyseur. C'est ainsi que pour des catalyseurs comprenant des quantités croissantes d'hydrazine, la conversion d'acrylonitrile en acrylamide passe par une valeur maximale. Au contraire, si on utilise le chlorhydrate d'hydroxylamine comme réducteur, le taux de conversion diminue avec l'augmentation de la quantité utilisée du réducteur. Toute ces considérations sont illustrées dans les essais du tableau I.

(Voir tableau page 4)

Tableau I

Effets du choix du réducteur et de la quantité utilisée

| ESSAI N° | REDUCTEUR | RELATION MOLAIRE REDUCTEUR / Cu | CONVERSION EN ACRYLAMIDE % (*) |
|----------|-----------|-------------------|-------------------|
| 1 | $NH_2OH.HCL$ | 0,50 | 25,9 % |
| 2 | $NH_2OH.HCL$ | 1,00 | 19,6 % |
| 3 | $NH_2OH.HCL$ | 1,50 | 15,4 % |
| 4 | $NH_2NH_2$ | 0,25 | 19,7 % |
| 5 | $NH_2NH_2$ | 0,50 | 26,1 % |
| 6 | $NH_2NH_2$ | 0,75 | 29,5 % |
| 7 | $NH_2NH_2$ | 1,50 | 13,3 % |

(*) Pour une réaction d'hydratation d'un mélange 50 : 50 en volume d'acrylonitrile/eau pendant 60 minutes à 90 °C.

Une des principales caractéristiques de l'invention est que les catalyseurs à base de cuivre et de vanadium sont obtenus à partir d'une solution d'ions cuivre II complexés par l'action d'un réducteur en présence du composé de vanadium.

Un autre avantage découle du fait que le catalyseur est préparé entièrement à température ambiante. A côté de l'avantage économique, il y a aussi un avantage technique important qui résout un des problèmes posés par l'état de la technique. On sait, en effet, que dans la réduction de composés de cuivre pour l'obtention de catalyseurs à base de ce métal, une augmentation de la température de réduction provoque la formation de catalyseurs moins actifs difficiles à séparer. Le tableau II ci-dessous, illustrant ce fait, montre que la réduction faite à température ambiante (essai n° 1) conduit à des catalyseurs plus actifs et plus faciles à décanter.

Tableau II

Influence de la température de réduction du composé de cuivre ($CuSO_4$) par l'hydroxylamine dans la conversion de l'acrylonitrile

| ESSAI N° | TEMPERATURE DE REDUCTION | CONVERSION MOYENNE | DECANTATION |
|----------|--------------------------|--------------------|-------------|
| 1 | 28°C (ambiante) | 19,6 % | facile |
| 2 | 50°C | 9,7 % | acceptable |
| 3 | 80°C | ---- | catalyseur pas de décantation |

4

Les catalyseurs ainsi obtenus doivent être conservés sous une atmosphère inerte afin de préserver leur réactivité et leur durée de vie.

Les catalyseurs à base de cuivre et de vanadium ainsi obtenus s'appliquent notamment aux réactions de préparation d'amides aliphatiques insaturés par hydratation des nitriles correspondants, tels que l'acrylonitrile et le méthacrylonitrile. L'efficacité des catalyseurs obtenus par le procédé selon l'invention permet leur utilisation dans de larges limites de proportions acrylonitrile/eau dans les mélanges de départ.

Les catalyseurs ainsi obtenus peuvent être utilisés plusieurs mois après leur préparation sans présenter de changements dans leurs caractéristiques physiques ou d'application dans l'hydratation catalytique des nitriles.

Une autre caractéristique importante du catalyseur obtenu selon la présente invention est le maintien de son activité au cours d'utilisations successives. Le tableau III ci-dessous illustre une partie d'une série d'essais faits à partir d'une seule quantité de catalyseur utilisée pour convertir l'acrylonitrile en acrylamide, sans renouvellement de la quantité de départ et sans l'utilisation de techniques ou de procédés pour régénérer cette quantité de départ mise dans le réacteur d'hydratation.

Tableau III

Maintien de l'activité du catalyseur

| ESSAI N° | TEMPS ECOULE PAR RAPPORT AU 1ER ESSAI | % TAUX DE CONVERSION D'ACRYLONITRILE |
|---|---|---|
| 1 | ---- | 41,1 % |
| 2 | 30 h | 48,1 % |
| 3 | 45 h | 49,5 % |
| 4 | 84 h | 49,9 % |
| 5 | 123 h | 49,0 % |
| 6 | 153 h | 47,6 % |
| 7 | 228 h | 51,3 % |

La présente invention a également pour objet le procédé d'hydratation de l'acrylonitrile en acrylamide en présence dudit catalyseur ci-dessus décrit.

Plus précisément, il s'agit d'un procédé d'hydratation en discontinu de solutions aqueuses d'acrylonitrile pour obtenir des solutions aqueuses d'acrylamide à 50 % en poids environ.

Industriellement, on prépare l'acrylamide par hydratation catalytique de l'acrylonitrile et on obtient, à la fin du procédé, l'acrylamide monomère sous la forme de cristaux ou de préférence sous forme de solutions aqueuses généralement à 50 % en poids ou en volume.

La plupart des procédés industriels pour l'obtention de solutions aqueuses d'acrylamide utilisent des méthodes continues ; quelques-uns utilisent des méthodes semi-continues. Ils ont tous pour but la production de tonnages élevés, de l'ordre de 10 000 tonnes annuelles.

Cependant, des problèmes techniques et économiques surviennent quand il s'agit de produire commercialement, à petite échelle ou dans des installations de petite taille, des solutions aqueuses d'acrylamide ; il en est ainsi par exemple lorsque l'on désire une production de quantités de l'ordre de quelques tonnes ou bien quand on envisage un marché consommateur de, par exemple, 3 ou 4 000 tonnes par an. Dans de telles situations, les procédés continus ou semi-continus ne sont pas économiquement viables, vu les investissements nécessaires, les coûts d'opération, la taille de l'installation, la rentabilité, etc. Un procédé du type discontinu (par « batch ») est alors très souhaitable. Mais on attend encore que ce procédé discontinu soit économique, qu'il présente des cycles d'opération courts, une grande productivité et que les solutions d'acrylamide obtenues soient d'une qualité du niveau de celles qui existent déjà sur le marché, obtenues par des procédés continus.

L'un des rares procédés brevetés pour l'obtention de solutions aqueuses d'acrylamide par une méthode discontinue est celui présenté par le brevet brésilien PI 7500916. Il propose une suite d'étapes qui comprend la suspension dans l'eau de particules d'un catalyseur au cuivre, l'addition par incréments de l'acrylonitrile dans la suspension aqueuse du catalyseur à la température de réaction (82 °C-149 °C), le maintien de cette température dans la zone de réaction, la décantation des particules catalytiques par l'arrêt de l'agitation, la récupération du produit de réaction et un nouveau chargement d'eau pour la répétition du cycle. D'après ce brevet, on peut se servir au moins 15 fois d'une même charge de catalyseur. Ce procédé présente de nombreux inconvénients.

Ainsi, d'après la revendication principale, le procédé est limité aux particules de catalyseur de diamètre moyen compris entre 50 microns et 2,5 mm. Des particules plus petites présentent des difficultés de sédimentation, tandis que les plus grandes provoquent des problèmes de décantation ainsi que de friction.

Il faut également remarquer qu'à la fin de l'addition de l'acrylonitrile on obtient des proportions eau/acrylonitrile comprises entre 75 : 25 (3 : 1) et 50 : 50 (1 : 1). Vu la haute température de la suspension aqueuse du catalyseur (82 °C-149 °C) au moment du départ de l'addition, l'introduction rapide de tout l'acrylonitrile à hydrater est impossible à cause des graves risques de réactions secondaires à cette température et d'explosions en fonction des pressions autogènes créées par la nature exothermique de la réaction d'hydratation de l'acrylonitrile. Si, d'une part, l'addition de nitrile en incréments proposée par le brevet brésilien évite les problèmes déjà nommés ci-dessus, d'un autre côté, elle introduit un inconvénient sérieux, celui du grand intervalle de temps nécessaire à l'addition de tout l'acrylonitrile dans le système (les exemples donnés dans le brevet mentionnent des temps d'addition supérieurs à 6 heures pour l'introduction de 105,7 kg de la nitrile dans l'exemple 1 et de 132,4 litres dans l'exemple 3).

Un autre inconvénient résulte de l'étape de maintien de la température dans la zone de réaction après l'addition de toute la charge de nitrile (étape C) où, d'après les exemples, les intervalles de temps sont de l'ordre de 7 heures.

Le procédé discontinu (par « batch ») décrit dans le brevet brésilien exige donc des intervalles de temps trop longs, ce qui pratiquement empêche son exploitation commerciale dans un marché de concurrence si l'on mesure la productivité en kg d'acrylamide produite par heure et par litre de réacteur.

Tous ces inconvénients, outre l'intérêt de la recherche pour l'obtention d'un procédé discontinu mais plus économique, plus efficace et plus productif, ont poussé la demanderesse au développement d'un procédé perfectionné pour l'obtention de solutions aqueuses d'acrylamide, procédé qui élimine les inconvénients de l'art antérieur et permet l'obtention de solutions sans polymères selon des cycles d'opération beaucoup plus rapides et où le nombre minimum de réactions qu'on peut réaliser avec une seule charge de départ de catalyseur est bien supérieur à celui des procédés actuels.

Le procédé d'hydratation en discontinu de l'acrylonitrile en acrylamide faisant l'objet de l'invention est caractérisé en ce que
— dans une première étape on fait réagir sous atmosphère inerte de l'eau et de l'acrylonitrile en présence du catalyseur au cuivre et au vanadium faisant l'objet de l'invention et d'un inhibiteur de polymérisation à une température comprise entre 80 et 110 °C, les quantités de réactifs correspondant à :
un rapport volumique acrylonitrile/eau allant de 0,05/1 à 2/1 ;
5 à 8 % en poids de catalyseur par rapport au milieu réactionnel ;
50 à 1 000 ppm d'inhibiteur de polymérisation par litre de mélange à réagir ;
— dans une deuxième étape, après séparation par décantation du catalyseur, on sépare l'acrylonitrile non réagi par distillation ;
— dans une troisième étape, on traite la solution avec du charbon actif pendant 10 à 60 minutes à une température allant de la température ambiante à 90 °C ;
— dans une quatrième étape, on élimine les éventuels ions métalliques résiduels par passage de la solution aqueuse d'acrylamide sur un lit de résines cationiques/anioniques.

Une caractéristique importante du procédé selon cette invention est qu'il permet l'emploi de mélanges acrylonitrile/eau de départ très riches en acrylonitrile ; il est en cela différent des autres procédés. On sait que les procédés continus par exemple, sont restreints aux proportions acrylonitrile/eau, généralement autour de la limite de « solubilité » (c'est-à-dire de la formation de solutions homogènes) de l'ordre de 7 g de nitrile/100 ml d'eau. Cette restriction vient du besoin de travailler avec des systèmes monophasiques surtout si le catalyseur est du type lit fixe. Cette proportion peut être un peu plus grande (d'environ 10 jusqu'à 30 %) si le catalyseur est en suspension ; mais, dans ce cas, une certaine sophistication des appareils est nécessaire pour qu'on obtienne une homogénéité relative entre le nitrile, l'eau et le catalyseur, dans une tentative d'améliorer le contact entre les composants de la masse réactionnelle et le catalyseur ainsi que pour obtenir une bonne productivité et empêcher des réactions parallèles indésirables. Dans le cas de procédés discontinus (par « batch »), comme le procédé du brevet brésilien ci-dessus cité, la condition de proportion 50 : 50 (1 : 1) représente une limite. Dans le procédé selon la présente invention, au contraire, cette proportion de 1 : 1 ne représente que la condition optimale, préférentielle, correspondant à une réalisation plus efficace et productive de l'invention.

On a constaté que le catalyseur mis en œuvre selon notre invention a une activité catalytique élevée

6

au départ et présente un comportement qui peut être considéré comme évolutif. Cela signifie que l'activité du catalyseur au cuivre et au vanadium non seulement ne diminue pas au cours des réactions successives mais encore présente une certaine augmentation. Cela se traduit, dans la réalisation pratique de l'invention, par l'obtention de temps d'hydratation plus courts au cours des réactions successives.

Un autre facteur important résulte de la combinaison des caractéristiques de longue durée de vie utile, de l'activité catalytique élevée et de l'aspect évolutif de cette activité catalytique. De ces caractéristiques découle alors un autre aspect important de l'invention. Ce procédé permet la réalisation d'au moins 80 réactions et généralement d'au moins 100 réactions sans qu'il soit nécessaire de régénérer le catalyseur, réadapter sa proportion, etc. Un avantage important est le fait que la qualité des solutions d'acrylamide obtenues reste invariable et compatible avec la qualité des meilleures acrylamides qui existent dans le marché et qui résultent de procédés industriels continus.

Dans la première étape du procédé selon la présente invention, l'hydratation catalytique de l'acrylonitrile est effectuée.

Les mélanges acrylonitrile/eau de départ peuvent être utilisés dans des proportions très variées. Bien que les proportions acrylonitrile/eau, riches en eau, répondent aussi aux objectifs de l'invention, le procédé se prête plus avantageusement aux proportions acrylonitrile/eau plus riches en nitrile, en raison de facteurs techniques et économiques. En effet, plus grande est la quantité d'eau mise en œuvre, plus grande sera l'énergie à fournir ; de même, plus grande est la quantité de nitrile mise en œuvre, plus grande sera l'énergie libérée puisque l'hydratation de l'acrylonitrile est une réaction exothermique. Les situations économiques les plus favorables seront celles où une moins grande quantité d'énergie à fournir est nécessaire et où davantage d'énergie thermique est libérée. Une conséquence de ces considérations apparaît clairement à l'homme de la technique en ce qui concerne les phases de déplacement de l'acrylonitrile et de l'eau en excès, et dans les étapes subséquentes du procédé selon l'invention.

L'addition des réactifs susdécrits ainsi que de l'inhibiteur de polymérisation nécessaire au procédé est faite simultanément à température ambiante, ce qui représente une économie de temps et d'énergie. Cette addition doit être faite sous une atmosphère inerte et l'eau utilisée est préalablement désaérée.

La réaction d'hydratation catalytique proprement dite est réalisée à une température comprise entre 80 et 110 °C pendant un intervalle de temps qui n'est pas supérieur à 4 heures.

La deuxième étape du procédé selon la présente invention comprend d'abord la séparation par décantation du catalyseur après l'arrêt de l'agitation. Aucune restriction quant à la taille des particules n'est à imposer audit catalyseur contrairement aux catalyseurs déjà connus du type cuivre de Raney.

On procède ensuite, à la séparation du nitrile non réagi par distillation. L'acrylonitrile non converti est ainsi récupéré et recyclé dans le procédé et la solution d'acrylamide est déchargée du réacteur.

Dans la troisième étape du procédé selon la présente invention la solution d'acrylamide libre d'acrylonitrile est traitée avec du charbon actif.

Au cours de ce traitement, on élimine la majorité des ions métalliques et des impuretés responsables des colorations des solutions d'acrylamide monomère.

La quatrième et dernière étape du procédé selon la présente invention est le passage de la solution d'acrylamide sur un lit mixte de résines cationiques/anioniques pour une éventuelle élimination d'ions métalliques résiduels.

Les deux dernières étapes susnommées contribuent à la haute qualité du produit car ainsi, libre de polymères et d'ions métalliques, ce produit final qui est une solution aqueuse d'acrylamide monomère à environ 50 % en poids, sert à toutes les finalités grâce à sa pureté.

Le catalyseur utilisé dans la première étape à base de cuivre et de vanadium, présente un rapport molaire Cu/V compris entre 6 : 1 et 200 : 1. On utilise de préférence des catalyseurs de cuivre et de vanadium dans lesquels le rapport molaire Cu/V est compris entre 16 : 1 et 65 : 1.

Comme le procédé est bien adapté à des proportions élevées en nitrile, les conditions préférentielles de réalisation de l'invention sont celles qui utilisent des proportions acrylonitrile/eau autour de 1 : 1 en volume. L'addition des réactifs susnommés est faite à froid et simultanément avec l'eau déminéralisée et sans air. L'acrylonitrile du commerce contient déjà généralement une certaine quantité d'inhibiteur de polymérisation de l'ordre de 30 à 50 ppm. Parmi les inhibiteurs on peut citer par exemple le sulfate ferreux heptahydraté, les quinones comme le méthyl éther de l'hydroquinone, l'hydroquinone et le tertiobutyl catechol, parmi d'autres. Le sulfate de fer heptahydraté peut être utilisé d'une manière préférentielle.

Après avoir introduit tous les réactifs dans le réacteur, sous une atmosphère inerte, on réchauffe le système jusqu'à une température comprise entre 80 et 110 °C et de préférence entre 85 et 95 °C. Le temps de réaction nécessaire pour l'hydratation n'est pas supérieur à environ 4 heures. Cependant, dans des conditions optimales et avec le procédé optimisé, des temps totaux inférieurs à 3 heures peuvent être obtenus sans de grands problèmes. A la fin de la réaction, on refroidit le système.

Dans la deuxième étape, avec le système à une température maximum d'environ 50 °C, on arrête l'agitation ; le catalyseur décante. On décharge la masse réactionnelle en laissant le catalyseur dans le réacteur pour être utilisé dans la réaction suivante.

La masse réactionnelle déchargée est transférée dans un autre réacteur ; on récupère ensuite l'acrylonitrile non converti et l'inhibiteur de polymérisation qui peuvent être recyclés dans le réacteur contenant le catalyseur. Ensuite, par élévation de la température, on évapore une quantité d'eau suffisante pour obtenir une concentration d'acrylamide monomère en eau autour de 50 % en poids.

Dans la troisième étape, on procède à l'élimination d'impuretés et d'ions métalliques contaminants par traitement avec du charbon actif. Cette étape est réalisée à une température qui va de la température ambiante jusqu'à environ 90 °C pendant un intervalle de temps de 10 à 60 minutes. Après la filtration, la solution est limpide, incolore et sans impuretés.

Dans la quatrième étape, d'éventuelles traces d'ions métalliques peuvent être éliminées. Le procédé selon l'invention est destiné à produire des solutions aqueuses d'acrylamide d'excellente qualité pour toutes les applications ; ainsi dans le cas où l'on désire polymériser l'acrylamide, des traces de métaux doivent être éliminées, même si elles proviennent d'inhibiteurs utilisés antérieurement ou du catalyseur utilisé dans la 1re étape. Cette dernière étape consiste alors dans le passage de la solution aqueuse d'acrylamide sur une colonne de résines mixtes, c'est-à-dire cationiques/anioniques. La résine cationique se présente sous la forme $H^+$ et la résine anionique se présente sous la forme $Cl^-$. La préparation de cette colonne est faite selon les techniques connues des spécialistes. Le passage de la solution par cette colonne est fait à température ambiante.

S'il est nécessaire à la fin du procédé d'ajuster le pH, cela pourra être fait avec une solution 0,1 N d'hydroxyde de sodium. De la même façon, s'il est désiré d'ajuster la concentration finale d'acrylamide à une valeur déterminée, dilution plus élevée par exemple, il suffit d'ajouter de l'eau déminéralisée selon la dilution demandée.

Les solutions aqueuses à 50 % en poids d'acrylamide obtenues par le procédé selon l'invention ne contiennent pas de polymères.

Le procédé d'obtention de solutions aqueuses d'acrylamide à 50 % en poids selon l'invention est très économique. La productivité en acrylamide est de l'ordre de 65 kg/h/m$^n$, avec une consommation d'acrylonitrile de l'ordre de 0,77 kg par kilo d'acrylamide produit.

Les taux de conversion de l'acrylonitrile peuvent varier de 40 à 80 % pour une proportion en volume voisine de 1 : 1 entre le nitrile et l'eau. On en déduit alors que, bien que le taux de conversion ne soit pas total, le fait de partir d'une proportion élevée d'acrylonitrile dans le mélange de départ, permet l'obtention d'une grande productivité par heure de réaction, par volume utile du réacteur et par jour.

Les exemples suivants sont donnés à titre indicatif et ne peuvent pas être considérés comme une limite du domaine et de l'esprit de l'invention.

## Exemple 1

Dans un réacteur en verre de 4 litres, taversé par un courant d'azote et contenant 2,5 litres d'eau déminéralisée, on dissout à température ambiante, sous agitation, 355 g de tartrate double de sodium et de potassium (sel de Rochelle). Le milieu est alcalinisé avec 160 ml d'une solution à 50 % en poids d'hydroxyde de sodium. Dans les mêmes conditions, on dissout dans le milieu 266 g de sulfate cuivreux pentahydraté, on additionne 164,9 g de vanadate d'ammonium et 76 g de chlorhydrate d'hydroxylamine. Par filtration, suivie de lavages à l'eau, on obtient 68,1 g de catalyseur.

Cette quantité de catalyseur est utilisée pour hydrater un mélange de 500 ml (403 g) d'acrylonitrile et 500 ml d'eau, ce qui correspond à 7,5 % de catalyseur par rapport à la masse réactionnelle de départ ; la réaction est effectuée pendant 1 heure à 90 °C, en présence d'une petite quantité (250 mg) d'un inhibiteur de polymérisation. On obtient à la fin une conversion de 24,3 % en acrylamide avec une sélectivité d'environ 100 %.

## Exemple 2

Dans les mêmes conditions que celles de l'exemple 1, on dissout dans l'eau 355 g du sel de Rochelle et on alcalinise le milieu avec 160 ml d'une solution à 50 % d'hydroxyde de sodium. Ensuite, on ajoute 266 g de sulfate cuivreux pentahydraté et 128 g de pentoxyde de vanadium en conservant les mêmes conditions réactionnelles. On fait la réduction des ions cuivre II à l'aide de 240,1 g d'hydrazine monohydratée à 22 %. Après filtration et lavage du précipité, on obtient 82,8 g du catalyseur. Cette quantité de catalyseur utilisée dans les mêmes conditions d'hydratation qu'à l'exemple 1, permet la conversion de 30,9 % d'acrylonitrile en acrylamide, la sélectivité étant de 100 % environ.

## Exemple 3

On prépare un catalyseur selon les conditions décrites dans l'exemple 2, en substituant l'hydrazine par 76 g de chlorhydrate d'hydroxylamine. Le catalyseur obtenu, appliqué à l'hydratation d'un mélange 1 : 1 en volume acrylonitrile/eau, donne une conversion de 22,5 % de nitrile en acrylamide avec une sélectivité de 100 %.

## Exemple 4

Le même catalyseur que celui préparé à l'exemple 3 est utilisé pour l'hydratation d'une solution aqueuse à 7 % en volume d'acrylonitrile. Après une heure de réaction à 90 °C, le taux de conversion est de 76,1 %.

## Exemple 5

Dans un réacteur en verre, de 4 litres, sous atmosphère d'azote et contenant 1,25 litre d'eau déminéralisée, on dissout à température ambiante et sous agitation 355 g de tartrate double de sodium et de potassium. On alcalinise le milieu avec 160 ml d'une solution à 50 % en poids d'hydroxyde de sodium. On dissout ensuite dans les mêmes conditions 107 g d'hydroxyde cuivreux puis on ajoute 12,8 g de pentoxyde de vanadium. La réduction du cuivre est faite à l'aide de l'addition de 125 ml d'une solution à 15 % d'hydrazine monohydratée ; à la fin de la séparation et du lavage du précipité, on obtient autour de 64 g de catalyseur sec. Cette masse de catalyseur, appliquée à l'hydratation d'un mélange 1 : 1 d'eau/acrylonitrile pendant 1 heure à 100 °C, montre une conversion en acrylamide de 26,8 %.

## Exemple 6

Dans un réacteur avec une capacité de 2,5 litres, muni d'un agitateur et traversé par un flux d'azote, on charge à une température ambiante 500 ml d'eau contenant 68 g de catalyseur à base de cuivre et vanadium préparé à l'exemple 1 (correspondant à un rapport Cu/V de 96/4) et 500 ml d'acrylonitrile qui contient 250 mg de sulfate ferreux heptahydraté comme inhibiteur de polymérisation. L'ensemble est alors réchauffé jusqu'à environ 95 °C et maintenu à cette température pendant à peu près 2 heures.

A la fin de cette phase d'hydratation, le système est refroidi jusqu'à environ 40 °C. Après arrêt de l'agitation, on sépare par décantation le catalyseur et on décharge la masse réactionnelle liquide dans un autre réacteur de 3 litres nanti d'une colonne de garnissage accouplée à un condenseur. Jusqu'ici, le temps total écoulé depuis le début de l'addition d'acrylonitrile, de l'eau et du catalyseur (1re phase) jusqu'à la décharge de la masse réactionnelle, est de 3 heures.

Ce deuxième réacteur est réchauffé et mis sous léger vide ; l'acrylonitrile non converti distille ; ensuite, on concentre la solution d'acrylamide par évaporation de l'eau, jusqu'à atteindre une concentration autour de 50 % en poids d'acrylamide en eau.

La solution obtenue, libre d'acrylonitrile, est traitée avec à peu près 1 % en poids de charbon actif pendant environ 30 minutes à une température comprise entre 30 °C et 90 °C.

Dans une quatrième et dernière étape, on élimine les éventuelles traces d'ions métalliques par le passage de la solution à travers une colonne mixte de résines cationiques/anioniques. La solution finale d'acrylamide monomère à 50 % en poids obtenue est limpide, incolore et libre de polymères, d'acrylonitrile et d'ions métalliques. La conversion obtenue est d'environ 48,1 %.

## Exemple 7

En se servant de l'installation et des conditions de l'exemple 6, on introduit au départ 68 g du catalyseur au cuivre et au vanadium avec de l'eau (500 ml) et de l'acrylonitrile (500 ml). Ensuite, 100 réactions successives sont effectuées avec cette même quantité de catalyseur mise au départ, sans aucune réactivation, addition, régénération ou traitement dudit catalyseur. Le tableau ci-dessous résume quelques valeurs obtenues tout au long des 100 réactions.

### Tableau IV

| N° DE LA REACTION | TEMPS DE REACTION (heures) * | % DE CONVERSION DE L'ACRYLONITRILE ** |
|---|---|---|
| 001 | 2,5 | 41,1 |
| 010 | 2,5 | 48,1 |
| 015 | 2,5 | 49,5 |
| 019 | 2,0 | 53,7 |
| 025 | 2,0 | 52,3 |
| 050 | 2,0 | 48,1 |

**0 145 553**

Tableau IV  (Suite)

| N° DE LA REACTION | TEMPS DE REACTION (heures) * | % DE CONVERSION DE L'ACRYLONITRILE ** |
|---|---|---|
| 071 | 2,0 | 49,1 |
| 075 | 2,0 | 56,0 |
| 085 | 2,0 | 50,9 |
| 099 | 2,0 | 50,4 |
| 100 | 2,0 | 50,4 |

(*) Le temps de réaction est compté à partir de la fin du chargement des réactifs (avant le début du réchauffement) jusqu'au début du refroidissement.

(**) Le pourcentage moyen de conversion au long des 100 réactions est de 46,9 %.

## Revendications

1. Procédé de préparation d'un catalyseur à base de cuivre et de vanadium pour l'hydratation de nitriles caractérisé en ce qu'il est réalisé à température ambiante sous atmosphère inerte et comporte les étapes suivantes :

a) mise en solution dans l'eau d'un agent complexant susceptible de complexer sous forme hydrosoluble les composés de cuivre II ;

b) alcalinisation de la solution obtenue par un hydroxyde de métal alcalin ;

c) mise en solution dans le milieu obtenu à l'étape b) d'un composé du cuivre II ;

d) mise en solution dans le milieu obtenu à l'étape c) d'un composé hydrosoluble du vanadium ;

e) mise en solution dans le milieu à l'étape d) d'un agent réducteur des ions cuivre II ;

f) et récupération du catalyseur formé après lavage du précipité obtenu ;

les quantités de réactifs mises en œuvres aux étapes a), b), c), d) et e) correspondant à :

— un rapport molaire agent complexant/composé du cuivre II allant de 2/1 à 10/1 ;

— un rapport molaire base/composé de cuivre II allant de 2/1 à 10/1 ;

— un rapport molaire cuivre II/vanadium d'au moins 6/1 ;

— une quantité d'agent réducteur allant de 100 % à 300 % de la quantité stoechiométrique.

2. Procédé selon la revendication 1 caractérisé en ce que l'agent complexant est l'acide éthylènediaminetétracétique, le tartrate double de sodium et de potassium, le chlorhydrate d'orthophénantroline, l'hexamétaphosphate de sodium, le citrate de sodium, la cuproïne.

3. Procédé selon la revendication 2), caractérisé en ce que l'agent complexant est du tartrate double de sodium et de potassium et en ce qu'il est mis en œuvre selon un rapport molaire agent complexant/composé du cuivre II allant de 1/1 à 3/1.

4. Procédé selon l'une quelconque des revendications précédentes, caractérisé en ce que la base est de l'hydroxyde de sodium en solution aqueuse à 5-50 % en poids.

5. Procédé selon l'une quelconque des revendications précédentes caractérisé en ce que le composé de cuivre II est du chlorure cuivreux dihydraté, du nitrate cuivreux hexahydraté, du sulfate cuivreux pentahydraté, de l'hydroxyde cuivreux.

6. Procédé selon l'une quelconque des revendications précédentes caractérisé en ce que le composé de vanadium est du pentoxyde de vanadium, du vanadate d'ammonium, du sulfate de vanadyle.

7. Procédé selon l'une quelconque des revendications précédentes caractérisé en ce que le rapport molaire cuivre II/vanadium va de 16/1 à 200/1.

8. Procédé selon l'une quelconque des revendications précédentes caractérisé en ce que l'agent réducteur est le borohydrure de sodium, l'hydrazine, l'hypophosphite de sodium, le chlorhydrate d'hydroxylamine.

9. Procédé d'hydratation en discontinu de l'acrylonitrile en acrylamide caractérisé en ce que :

10

— dans une première étape on fait réagir sous atmosphère inerte de l'eau et de l'acrylonitrile en présence du catalyseur au cuivre et au vanadium obtenu selon le procédé faisant l'objet de l'une quelconque des revendications précédentes et d'un inhibiteur de polymérisation à une température comprise entre 80 et 110 °C, les quantités de réactifs correspondant à :

un rapport volumique acrylonitrile/eau allant de 0,05/1 à 2/1 ;

5 à 8 % en poids de catalyseur par rapport au milieu réactionnel ;

50 à 1 000 ppm d'inhibiteur de polymérisation par litre de mélange à réagir ;

— dans une deuxième étape, après séparation par décantation du catalyseur, on sépare l'acrylonitrile non réagi par distillation ;

— dans une troisième étape, on traite la solution avec du charbon actif pendant 10 à 60 minutes à une température allant de la température ambiante à 90 °C ;

— dans une quatrième étape, on élimine les éventuels ions métalliques résiduels par passage de la solution aqueuse d'acrylamide sur un lit de résines cationiques/anioniques.

10. Procédé selon la revendication 9) caractérisé en ce que le catalyseur à base de cuivre et de vanadium présente un rapport molaire cuivre/vanadium allant de 16/1 à 65/1.

11. Procédé selon la revendication 9 ou 10 caractérisé en ce que le rapport volumique acrylonitrile/eau est voisin de 1/1.

12. Procédé selon l'une quelconque des revendications 9 à 11 caractérisé en ce que la 1re étape est réalisée à une température comprise entre 85 et 95 °C.

## Claims

1. A process for the preparation of a catalyst based on copper and vanadium for the hydration of nitriles characterised in that it is produced at ambient temperature in an inert atmosphere and comprises the following steps :

a) dissolving in water a complexing agent capable of complexing copper II compounds in water-soluble form ;

b) alkalinising the solution produced by an alkali metal hydroxide ;

c) dissolving a compound of copper II in the medium obtained in step b) ;

d) dissolving a water-soluble compound of vanadium in the medium produced in step c) ;

e) dissolving a reducing agent for the copper II ions in the medium from step d) ;

f) and recovering the catalyst formed after washing of the precipitate obtained ;

the amounts of reactants used in steps a), b), c), d) and e) corresponding to :

— a complexing agent/copper II compound molar ratio ranging from 2/1 to 10/1 ;

— a base/copper II compound molar ratio ranging from 2/1 to 10/1 ;

— a copper II/vanadium molar ratio of at least 6/1 ; and

— an amount of reducing agent ranging from 100 % to 300 % of the stoichiometric amount.

2. A process according to claim 1 characterised in that the complexing agent is ethylenediaminetetracetic acid, the double tartrate of sodium and potassium, orthophenantrolinehydrochloride, sodium hexametaphosphate, sodium citrate or cuproine.

3. A process according to claim 2 characterised in that the complexing agent is double tartrate of sodium and potassium and that it is used in a complexing agent/copper II compound molar ratio ranging from 1/1 to 3/1.

4. A process according to any one of the preceding claims characterised in that the base is sodium hydroxide in 5-50 % by weight aqueous solution.

5. A process according to any one of the preceding claims characterised in that the copper II compound is dihydrated cuprous chloride, hexahydrated cuprous nitrate, pentahydrated cuprous sulphate and cuprous hydroxide.

6. A process according to any one of the preceding claims characterised in that the vanadium compound is vanadium pentoxide, ammonium vanadate or vanadyl sulphate.

7. A process according to any one of the preceding claims characterised in that the copper II/vanadium molar ratio ranges from 16/1 to 200/1.

8. A process according to any one of the preceding claims characterised in that the reducing agent is sodium borohydride, hydrazine, sodium hypophosphite or hydroxylamine hydrochloride.

9. A process for the discontinuous hydration of acrylonitrile to acrylamide characterised by :

— in a first step, reacting in an inert atmosphere water and acrylonitrile in the presence of the copper and vanadium-bearing catalyst produced by the process according to any one of the preceding claims and a polymerisation inhibitor at a temperature of from 80 to 110 °C, the amounts of reactants corresponding to :

an acrylonitrile/water ratio by volume ranging from 0.05/1 to 2/1 ;

from 5 to 8 % by weight of catalyst with respect to the reaction medium ; and

from 50 to 1 000 ppm of polymerisation inhibitor per litre of mixture to be reacted,

— in a second step, after separation by settlement of the catalyst, separating the unreacted acrylonitrile by distillation ;

**0 145 553**

— in a third step, treating the solution with activated carbon for from 10 to 60 minutes at a temperature ranging from ambient temperature to 90 °C ; and

— in a fourth stage, removing any residual metallic ions by passing the aqueous acrylamide solution over a bed of cationic/anionic resins.

10. A process according to claim 9 characterised in that the copper and vanadium-based catalyst has a copper/vanadium molar ratio ranging from 16/1 to 65/1.

11. A process according to claim 9 or claim 10 characterised in that the acrylonitrile/water ratio by volume is close to 1/1.

12. A process according to any one of claims 9 to 11 characterised in that the first step is carried out at a temperature of between 85 and 95 °C.


**Patentansprüche**

1. Verfahren zur Herstellung eines Katalysators auf Basis von Kupfer und Vanadin zur Hydratisierung von Nitrilen, dadurch gekennzeichnet, daß sie bei Raumtemperatur in einer Inertgasatmosphäre durchgeführt wird und die folgenden Schritte enthält :

a) Auflösen eines Komplexierungsagens, das geeignet ist, Kupfer (II)-Verbindungen in wasserlösliche Form zu bringen, in Wasser ;

b) Alkalisierung der erhaltenen Lösung mit einem Alkalimetallhydroxid ;

c) Auflösen einer Kupfer (II)-Verbindung in dem in Stufe b) erhaltenen Medium ;

d) Auflösung einer wasserlöslischen Vanadium-Verbindung in

e) Auflösung eines Reduktionsagens für Kupfer (II)-Ionen in dem in Stufe d) erhaltenen Medium ;

f) und, nach Wachsen des erhaltenen Niederschlags Gewinnung des gebildeten Katalysators, wobei die Mengen der in den Stufen a), b), c), d) und e) eingesetzten Reagenzien entsprechen :

— einem Molverhältnis Komplexierungsagens/Kupfer-(II)-Verbindung, das zwischen 2/1 und 10/1 liegt ;

— ein Molverhältnis Base/Kupfer (II)-Verbindung, das zwischen 2/1 und 10/1 liegt ;

— ein Molverhältnis Kupfer (II)/Vanadium von mindestens 6/1 ;

— einer Reduktionsmittelmenge, die zwischen 100 und 300 % der stöchiometrischen Menge liegt.

2. Verfahren nach Anspruch 1, dadurch gekennzeichnet, daß das Komplexierungsmittel Ethylendiamintetraessigsäure, das Doppeltartrat von Natrium und Kalium, Orthophenantrolinchlorhydrat, Natriumhexametaphosphat, Natriumcitrat, Cuproin ist.

3. Verfahren nach Anspruch 2, dadurch gekennzeichnet, daß das Komplexierungsmittel das Doppeltartrat von Natrium und Kalium ist und das es in einem Molverhältnis Komplexbildner/Kupfer (II)-Verbindung von 1/1 bis zu 3/1 eingesetzt wird.

4. Verfahren nach einem der vorhergehenden Ansprüche, dadurch gekennzeichnet, daß die Base Natriumhydroxid in 5-50 gew.-%iger, wässriger Lösung ist.

5. Verfahren nach einem der vorhergehenden Ansprüche, dadurch gekennzeichnet, daß die Kupfer (II)-Verbindung Kupferchlorid-Dihydrat, Kupfernitrat-Hexahydrat, Kupfersulfat-Pentahydrat, Kupferhydroxid ist.

6. Verfahren nach einem der vorhergehenden Ansprüche, dadurch gekennzeichnet, daß die Vanadiumverbindung Vanadiumpentoxid, Ammoniumvanadat, Vanadylsulfat ist.

7. Verfahren nach einem der vorhergehenden Ansprüche, dadurch gekennzeichnet, daß das Molverhältnis Kupfer (II)/Vanadin von 16/1 bis 200/1 geht.

8. Verfahren nach einem der vorhergehenden Ansprüche, dadurch gekennzeichnet, daß das Reduktionsmittel Natriumborhydrid, Hydrazin, Natriumhypophosphit, Hydroxylamin-Chlorhydrat ist.

9. Verfahren zur diskontinuierlichen Hydratisierung von Acrylnitril zu Acrylamid, dadurch gekennzeichnet, daß :

— in einer ersten Stufe in einer Inertgasatmosphäre Wasser und Acrylnitril in Gegenwart des Kupfer- und Vanadin-Katalysators, der nach dem Verfahren erhalten wurde, das den Gegenstand eines jeden der vorhergehenden Ansprüche darstellt, und eines Polymerisationsinhibitors bei einer Temperatur zwischen 80 und 110 °C zur Reaktion gebracht werden, wobei die Mengen der Reaktanten entsprechen :

einem Volumverhältnis Acrylnitril/Wasser von 0,05/1 bis 2/1 ;

5 bis 8 Gew.-% Katalysator bezogen auf das Reaktionsmedium ;

50 bis 1 000 ppm Polymerisationsinhibitor pro Liter Reaktionsmischung

— in einer zweiten Stufe, nach Abtrennung des Katalysators durch Dekantieren man das nicht abreagierte Acrylnitril durch Destillation entfernt

— man in einer dritten Stufe die Lösung 10 bis 60 Minuten lang mit Aktivkohle bei einer Temperatur von Raumtemperatur bis 90 °C behandelt ;

— man in einer vierten Stufe gegebenenfalls vorhandene restliche Metallionen durch Durchlaufen der wässrigen Acrylamid-Lösung ein Bett von kationischen/anionischen Harzen entfernt.

10. Verfahren nach Anspruch 9, dadurch gekennzeichnet, daß der Katalysator auf Basis von Kupfer und Vanadin ein Molverhältnis Kupfer/Vanadin von 16/1 bis 65/1 aufweist.

12

**0 145 553**

11. Verfahren nach den Ansprüchen 9 oder 10, dadurch gekennzeichnet, daß das Volumenverhältnis Acrylnitril/Wasser in der Nähe von 1/1 liegt.

12. Verfahren nach einem der Ansprüche 9 bis 11, dadurch gekennzeichnet, daß die erste Stufe bei einer Temperatur zwischen 85 und 95 °C durchgeführt wird.